(19) Europäisches Patentamt
European Patent Office
Office européen des brevets

(11) Publication number: **0 123 146**
**B1**

(12) **EUROPEAN PATENT SPECIFICATION**

(45) Date of publication of patent specification: **23.05.90**

(21) Application number: **84103220.4**

(22) Date of filing: **23.03.84**

(51) Int. Cl.[5]: **C 07 D 417/00,** A 61 K 31/47
// C07D215/46

(54) **Compound with antiinflammatory activity, process for preparation thereof and pharmaceutical compositions therefrom.**

(30) Priority: **06.04.83 IT 2046883**

(43) Date of publication of application:
**31.10.84 Bulletin 84/44**

(45) Publication of the grant of the patent:
**23.05.90 Bulletin 90/21**

(84) Designated Contracting States:
**AT BE CH DE FR GB IT LI LU NL SE**

(56) References cited:
**DE-A-2 847 792**

**Pharmazeutische Stoffliste (1987), pp. 267-268**

**The file contains technical information submitted after the application was filed and not included in this specification**

(73) Proprietor: **CHIESI FARMACEUTICI S.p.A.**
**Via Palermo, 26/A**
**I-43100 Parma (IT)**

(72) Inventor: **Quadro, Giuseppe**
**Via Pisacane 34/A**
**I-20100 Milano (IT)**

(74) Representative: **Bianchetti, Giuseppe**
**Studio Consulenza Brevettuale Via Rossini, 8**
**I-20122 Milan (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 123 146 B1

**Description**

The present invention relates to a compound of formula (I):

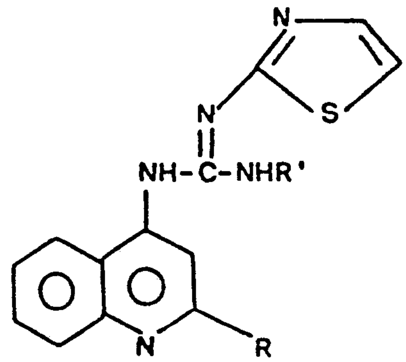

I

in which:

R represents a methylthio group;

R′ represents a cyclohexyl group.

The present invention relates also to pharmaceutically acceptable salts of compound I with organic acids (such as acetic, succinic, tartaric, citric acid) or inorganic acids (such as hydrochloric, hydrobromic, sulfuric acid).

In published German Patent Application No. 2847792 analogous N-4-quinoline-guanidine derivatives are described in which, however, R never represents a thioalkyl group.

Moreover, even if $C_1$—$C_6$ alkoxy groups are comprised in the numerous meanings assigned to the general formula, such groups are never considered as preferred substituents.

The compound of formula I has excellent antiinflammatory, analgesic and antipyretic activities and is a potent inhibitor of the activity of prostaglandin-synthetases.

It has now been surprisingly found that the compound of formula I shows advantageous therapeutic characteristics with respect to known antiinflammatory agents and to compounds described in the above cited German Patent Application.

The compound of the invention may be prepared with a process which comprises the following steps:

1. reacting a 4-aminoquinoline (II) with an isocyanate (III);
2. subsequent dehydration of the resulting urea to carbodiimide, using triphenylphosphine and triethylamine in carbon tetrachloride;
3. reacting the resulting compound with 2-aminothiazole.

The reaction scheme is reported hereinbelow, in which R and R′ have the above mentioned meanings.

SCHEME I

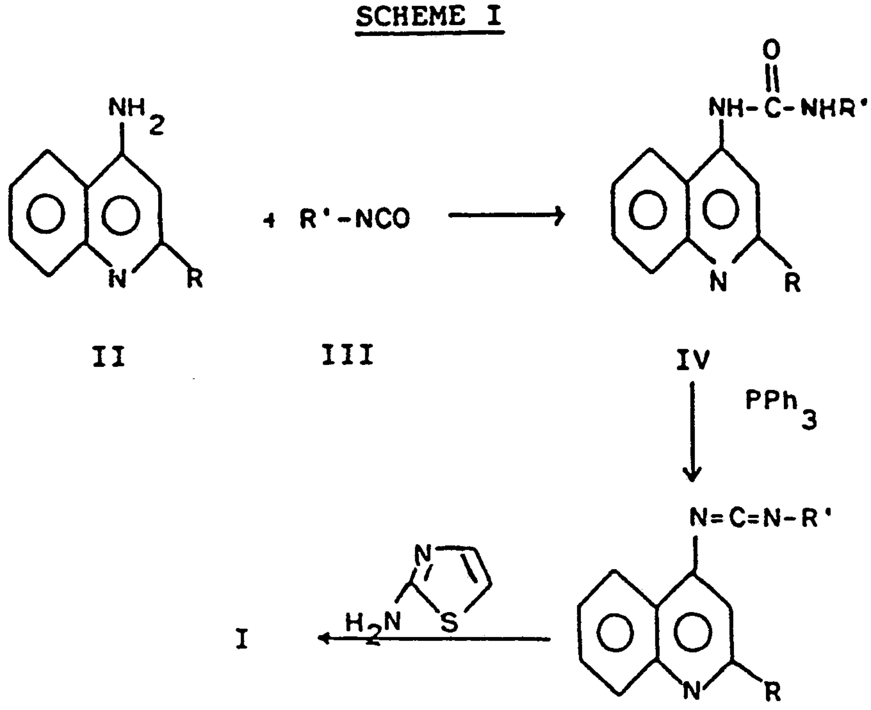


II III IV V I

The starting 4-aminoquinolines, in which R is a thioalkyl group, may be suitably prepared according to the following scheme, in which R'' represents a methyl group:

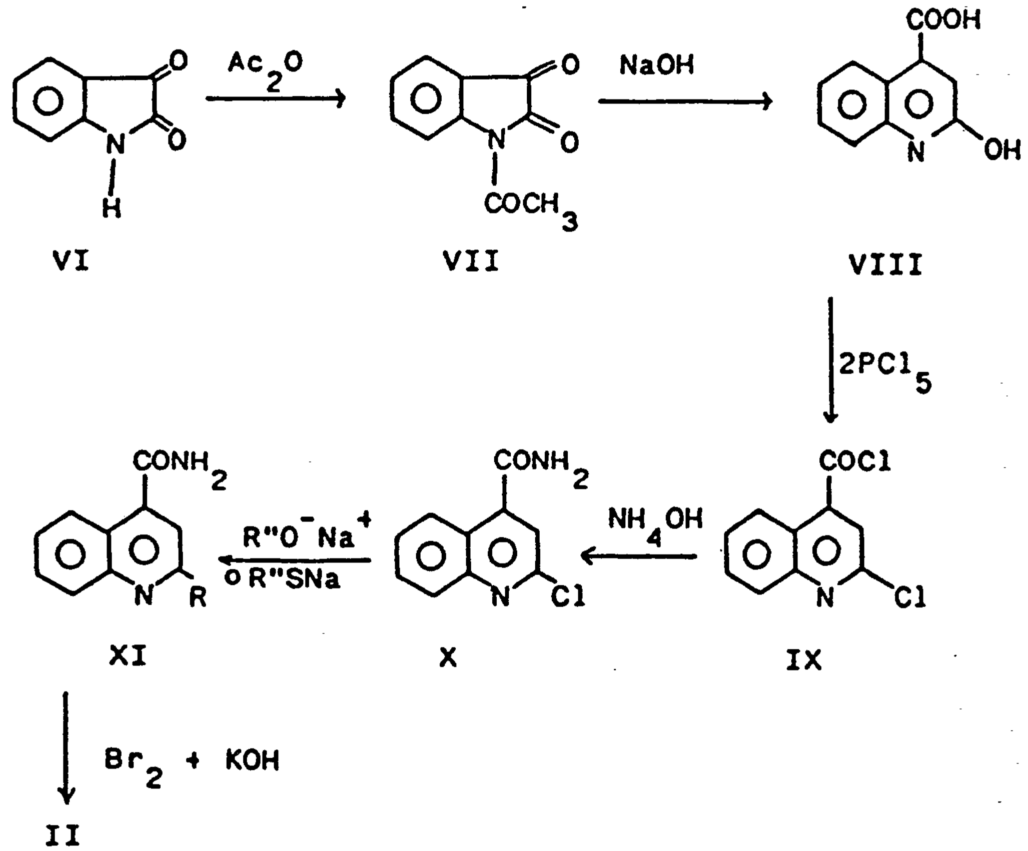


VI VII VIII IX X XI II

The 2-hydroxy-4-quinolinecarboxylic acid (VIII), obtained by rearrangement of N-acetyl-isatin (VII), is transformed into the 2-chloro-4-quinolinecarboxylic acid amide by treatment with $PCl_5$ and $NH_4OH$. The nucleophilic substitution of the chlorine atom at 2-position and the Hoffmann reaction give starting compouinds (II).

The preparation of the compound of the invention is further illustrated by the following examples.

Preparation 1

a) N-Acetyl-isatin (VII)

50 g (0.34 moles) of isatin in 120 ml of acetic anhydride was heated to reflux for 30 minutes, with stirring.

The reaction mixture was kept for a night at room temperature: acetyl isatin precipitated in form of a yellow residue, which was recovered and washed with acetic anhydride.

After evaporation of the acetic anhydride, 20 g of a solid residue was obtained, which was acetylisatin (as it appeared by T.L.C.).

The two residues were pooled, suspended in 200 ml of toluene and kept under stirring for 10 minutes, filtered and dried at 70°C.

The resulting orange solid was free from acetic anhydride.

M.p.: 130—133° [Lit. 140°C].

Yield: 84% (54 g).

T.L.C. ($PhCH_3$/AcOEt = 75/25): slightly impure for isatin.

b) 2-Hydroxy-4-quinolinecarboxylic acid (VIII)

25.2 g (0.286 × 2.2 moles) of NaOH tablets was dissolved in 500 ml of water. The solution was heated to reflux, then 54 g (0.286 moles) of compound (VII) was added, in 4 portions.

The reaction mixture was refluxed for 1 hour. After cooling and acidification with concentrated HCl, a yellow-orange precipitate was obtained, which was recovered by filtration. As it resulted slightly impure for the presence of isatin (checking by T.L.C.), such precipitate was poured in aceton, stirred for about 10 minutes (isatin is soluble in aceton while VIII is'nt) and filtered obtaining a yellow solid.

M.p.: 250°C [Lit. >340°C].

Yield: 30.9 g (57%).

T.L.C. ($PhCH_3$/AcOEt: 75/25): single spot — Rf = O.

c) 2-Chloro-4-quinolinecarboxylic acid chloride (IX)

23 g (0.122 moles) of compound (VIII) and 55.7 g (0.122 × 2 moles + 10% excess) of $PCl_5$ were heated in oil-bath to 140—150°C for 1 hour, under stirring.

The produced $POCl_3$ was evaporated off, and a brownish residue was obtained which was crystallized from n-hexane, giving an almost colourless residue.

M.p.: 85—87°C [Lit. 89°].

Yield: 27.8 g (quantitative on the crude product).

T.L.C. ($PhCH_3$/AcOEt: 75/25): single spot.

d) 2-Chloro-4-quinolinecarboxylic acid amide (X)

200 ml of a 32% $NH_4OH$ aqueous solution was cooled to 5°C; then 27.8 g (0.123 moles) of compound (IX) dissolved in 100 ml of toluene was added dropwise, with stirring, keeping the temperature between 5 and 10°c.

After completion of the addition, the reaction mixture was stirred for 10 minutes, then the precipitated amide was filtered off, giving a solid residue which was washed with water, filtered and finally crystallized from EtOH to give compound (I) in the form of a nearly colourless crystalline solid residue.

M.p.: 250—252°C [Lt. 238—239°C].

Yield: 23.4 g (92%).

T.L.C. ($PhCH_3$/AcOEt/triethylamine = 24/75/1): single spot.

e) 2-Ethoxy-4-quinolincarboxylic acid amide (XI)

Sodium ethylate was prepared by dissolving 1.25 g (0.048 moles + 10% excess) of metallic Na in 120 ml of anhydrous ethanol.

10 g (0.048 moles) of (X) was added thereto and the mixture was heated to reflux for 4 hours.

The solvent was evaporated and the obtained residue was washed with water and filtered. The product was then crystallized from ethanol obtained a nearly colourless crystallin solid (I).

M.p.: 206—208°C.

Yield: 9.2 g (88%).

T.L.C. (EtOH/AcOEt/triethylamine = 25/75/1): single spot.

I.R. (nujol): in agreement.

N.M.R. ($C_3D_6O$): in agreement.

f) 2-Ethoxy-4-aminoquinoline (II)

10 g (0.178 moles) of KOH was dissolved in 200 ml of water. The solution was cooled to 0°C, then 1.83 ml (0.0324 + 10% excess) of $Br_2$ was added. After about 10 minutes 7 g (0.0324 moles) of compound (XI) was added.

The reaction mixture was heated to 90—95°C for 4 hours, then it was cooled and extracted with ethyl ether. The ether phase was washed with water, dried over $Na_2SO_4$ and the solvent was evaporated off. The resulting residue was washed with diisopropyl ether and filtered, to give 5 g of a crude product still containing non reacted amide, which was eliminated by dissolving the solid in $HCl/H_2O$ = 1/1, to precipitate the concentrated amine hydrochloride which is filtered and dissolved in warm water.

The pH was adjusted to 10 with concentrated NaOH to precipitate compund (II) as free base, which was purified by column chromatography (150 g of $SiO_2$, eluent $PhCH_3$/AcOEt = 75/25), to eliminate the N,N'-bis-[2-ethoxy-4-aminoquinoline]-urea present as main by-product from the Hoffmann degradation reaction.

The pure amine of formula (II) was obtained.

M.p.: 128—130°C (Lit. 127°).

Yield: 3 g (49%).

I.R. Spectrum (nujol): in agreement.

Following the same procedure as described in Points e) and f), but carrying out the reaction in the presence of methylmercaptan 2-methylthio-4-aminoquinoline was obtained (m.p. 103—105°C).

Example 1

N-Cyclohexyl-N''-4-(2-methylthioquinolyl)-N'-2-thiazolylguanidine (I)

a) N-Cyclohexyl-N''-4-(2-methylthioquinoline)urea (IV)

1.81 g (0.034 moles + 10% excess) of 50% NaH was suspended in 100 ml anhydrous toluene and 20 ml anhydrous DMSO. 6.5 g (0.034 moles) of 4-amino-2-methylthioquinoline was added, then 5.64 ml (0.034 moles + 30% excess) of cyclohexylisocyanate was added dropwise.

The reaction mixture was heated to 80—90°C in oil-bath for 8 hours; the solvent was evaporated off, the residue was treated with water and the pH was adjusted to 5 with concentrated $CH_3COOH$. The resulting pale yellow residue (IV) was filtered and dried at 70°C.

M.p.: 207—209°C.

Yield: 10.6 g (98%).

T.L.C. ($PhCH_3$/AcOEt = 50/50): single spot.

N.M.R. (DMSO): in agreement.

The resulting urea was crystallized in the anhydrous form (Karl-Fisher Test: negative).

b) N'-Cyclohexyl-N''-4-(2-methylthioquinoline)carbodiimide (V)

10.6 g (0.034 moles) of IV and 10.05 g (0.038 moles) of $Ph_3P$ were suspended in 100 ml anhydrous $CH_2Cl_2$, and 3.4 ml (0.035 moles) of $CCl_4$ and 5 ml (0.036 moles) of triethylamine were added.

The reaction mixture was refluxed for 5 hours, after which the solvent was evaporated off and the residue was extracted with hot toluene. The insoluble triethylamine hydrochloride was filtered on Celite® and the solvent was evaporated off, to give a thick brown oil (V).

Yield: (quantitative of the crude product) 12 g.

T.L.C. ($PhCH_3$/AcOEt = 50/50): impure for triphenyl phosphine.

c) N-Cyclohexyl-N''-4-(2-methylthioquinolyl)-N'-2-thiazolylguanidine (I)

To a solution of 12 g (0.034 theoretical moles) of crude V in 100 ml of anhydrous toluene, 3.4 g (0.034 moles) of 2-aminothiazole was added.

The reaction mixture was refluxed for 7 hours, after which the solvent was evaporated off and the residue was chromatographed on 300 g of $SiO_2$ (eluent $PhCH_3$/AcOEt = 9/1), to give compound I as the free base, in the form of an orange oil (5.3 g).

The compound was dissolved in anhydrous EtOH and subjected to bubbling with gaseous HCl, to give the corresponding hydrochloride.

The ethanol was distilled off and the residue was treated with hot ethyl acetate and filtered, to give a solid yellow product.

M.p.: 208—210°C.

Yield: 4.5 g hydrochloride (31% on theoretical moles).

T.L.C. ($PhCH_3$/AcOEt = 50/50): single spot.

N.M.R. ($CDCl_3$): in agreement.

Elemental analysis: for $C_{20}H_{23}N_5O_2.HCl$ (M.W. = 433.5)

| | C | H | N |
|---|---|---|---|
| % calculated: | 55.36 | 5.54 | 16.15 |
| % found: | 54.35 | 5.67 | 16.00 |

The compound being the object of the present invention has been characterized from the toxico-pharmacological point of view.

In the following description the compound will be respectively marked as I (N-cyclohexyl-N''-4-(2-methylthioquinolyl)-N'-2-thiazolyl-guanidine).

As reference compounds, timegadine (structurally related compound previously described in the German Application No. 2847792 of Leo Pharmaceutical) and indomethacin were used.

Acute toxicity

The toxicity for single administration has been determined by the oral route in IVa:NMRI (SPF) fasted male mice, with water ad libitum, 18 hours before the experiment. The approximate $LD_{50}$ values, determined by interpolation on Probits® paper, are reported on Table 1.

TABLE 1

| Compound | $LD_{50}$ approx mg/kg |
|---|---|
| I | 1000 |
| Timegadine | 1000 |
| Indomethacin | 9.7[1)] |

[1)] Actual value, previously determined.

Antiinflammatory activity

It has been determined by the test of the plantar oedema by carrageenin in CrL:CD (SD) male rats, weighing 150—170 g preventively subjected to an acclimation period and fasted with water ad libitum 18 hours before the start of the test.

The activity of the compounds under exam, administered at different doses by oral route, has been evaluated by measuring the protection provided against the development of the oedema induced in the rat's paw by injecting (after one hour from the active principle administration), 0.1 ml of 1% carrageenin in saline solution in the subplantar aponeurosis of the rear right paw, according to the Winter method (Winter C. a. et al. Proc. soc. Exp. Biol. Med., 111, 544, 1962).

The results have been expressed both as $ED_{50}$ values determined in correspondence of the activity peak on the log. regression dose-% inhibition on the oedema development straight-line, and as $ED_{30}$ values calculated o the dose-percent inhibition log. regression straight lines, in comparison with the controls, on

the oedema development determined as mean AUC value (area under the curve representing the development of th paw's volume in time). The results are reported in Table 2.

TABLE 2

Antiinflammatory activity determined in the plantar oedema test by carrageenin in the rat, by oral administration. Statistical significance: *P<0.05; **P<0.01

| | | % inhibition oedema development as $ED_x$ values | | | | | |
|---|---|---|---|---|---|---|---|
| | | 2h | | 3h | | AUC | |
| Compound | Dose (mg/kg) | % inhib. | $ED_{50}$ (mg/kg) | % inhib. | $ED_{50}$ (mg/kg) | % inhib. | $ED_{50}$ (mg/kg) |
| | 100 | 71*** | | 61** | | 49* | |
| I | 30 | 52** | 30 | 34** | 62 | 23* | 40 |
| | 10 | 29* | | 5* | | 3 | |
| | 100 | 59** | | 52** | | 41** | |
| Timegadne | 30 | 55** | 34 | 34** | 89 | 29** | 37 |
| | 10 | 33** | | 20 | | 14 | |
| | 10 | 63** | | 53** | | 41** | |
| Indometacine | 3 | 36** | 5.2 | 26** | 9.1 | 19* | 5.3 |
| | 1 | 25** | | 9 | | 6 | |

Analgesic activity

Male mice weighing 19—22 g, housed in normal conditions, fasted for 18 hours and with free access to water, have been used.

The activity of the compounds under exam, administered by oral route at different doses, by gastric tube, suspended in 5% maize starch and in the constant volume of 10 ml/kg, has been evaluated by measuring the protection provided against a typical syndrome (writhing) induced by intraperitoneal injections of 0.1 ml/10 g body weight of a phenylquinone aqueous solution (0.02% in 5% ethanol) according to a modified experimental model (Arzneim. Forsch. 31(1), 87, 1981) in comparison with the one reported in literature by Siegmund (J. Pharmacol. Exp. Ther. 119, 184, 1957).

The controls received the vehicle only.

The $ED_{50}$ values determined from the dose-response curves are reported in Table 3.

TABLE 3

Analgesic activity determined in the phenylquinone "writhings" test in the mouse, by oral administration. Statistical significance: *P<0.05; **P<0.01

| Compound | Dose (mg/kg) | N. animals | N. writhings (1 hour) | % inhib. Vs control. ($\overline{X}$ ± E.S.) | $ED_{50}$ (mg/kg) | PR |
|---|---|---|---|---|---|---|
| Controls vehicle | — | 9 | 34 ± 4 | — | | |
| | 50 | 6 | 8 ± 4** | 78 | | |
| I | 15 | 7 | 20 ± 7* | 46 | 15.3 | 1 |
| | 5 | 6 | 27 ± 7 | 27 | | |
| | 50 | 6 | 8 ± 5** | 78 | | |
| Timegadine | 15 | 6 | 22 ± 5* | 41 | 15.3 | 1 |
| | 5 | 6 | 25 ± 8 | 32 | | |

Gastrolesive activity

The gastrolesive activity has been assayed in rats fasted for 18 h by means of macroscopic exam of the gastric mucosa after 7 hours from the administration of the substances under exam.

The animals having extension of the gastric lesions (sum of the largest diameters) ≥ 1 mm have been considered as positive.

For each treatment the log. regression straight line of the dose-mm of ulceration (single value for each animal) have been determined.

On the ground of these straight lines the $UD_0$ values, i.e. the maximum dose for which lesions are not expected, have been calculated for each compound.

From the $UD_0/ED_{50}$ ratio (antiinflammatory activity values relative to the AUCs as above defined) it has been possible to determine the therapeutic index for compound I in comparison with indomethacin.

The set of these results is shown in Table 4.

TABLE 4

Gastrolesive activity in the rat after 7 hours from the oral administration and determination of the absolute and relative therapeutic index of the compound I in comparison with timegadine and indomethacin, defined from the $UD_0$ (gastrolesivity value)/ $ED_{50}$ (antiinflammatory activity value) ratio.

| Compound | Antiinflammatory activity $ED_{50}$ (mg/kg) | Gastrolesive activity $UD_{50}$ (mg/kg) | T.I. ($UD_0/ED_{50}$) | Relative T.I. |
|---|---|---|---|---|
| I | 30 | 13.7 | 0.46 | 1.84 |
| Timegadine | 34 | 6.8 | 0.2 | 0.8 |
| Indomethacin | 5.2 | 1.3 | 0.25 | 1 |

T.I. = Therapeutic index.

The present invention refers also to all the industrially applicable aspects connected with the use of the compound of formula I in therapy as an antiinflammatory, analgesic and antipyretic agent.

The present invention refers also to pharmaceutical compositions containing as active principle the compound of formula (I), as above defined, as such or in form of a pharmaceutically acceptable salt, in admixture with at least one pharmaceutically acceptable excipient.

The composition can be administered by oral, rectal, parenteral or topic route, respectively in form of capsules, tablets or similar formulations, suppositories, vials, cream or gels.

For the preparation of pharmaceutical formulations for the oral administrations in unit dose, the active principle can be mixed with a solid powdered excipient such as lactose, saccharose, sorbitole, mannitole; potatoes, cereals of maize starch or amylopectine, a cellulose or gelatine derivative, and it can moreover contain lubricants such as talc, magnesium or calcium stearate, polyethyleneglycol or silica.

The tablets can be differently coated according to well known method in the pharmaceutical practice. Hard gelatine capsules can contain granulates of the active principle together with solid, powdered excipients such as lactose, saccharose, sorbitole, mannitole, starches (of the above mentioned kind), cellulose or gelatine derivative, and they can also contain stearic acid or magnesium stearate or talc.

Unit doses for rectal administrations can be in form of suppositories containing the active principle in combination with a neutral fatty carrier (for instance glycerides of fatty acids) or with hydrosoluble or self-emulsifiable (for instance, polyethyleneglycols mixture) excipients.

For injectable formulations for parenteral administration the excipients can be a pharmaceutically acceptable sterile liquid such as water or an aqueous solution of polyvinylpyrrolidone or even an oil such as peanut oil and optionally a stabilizing and/or buffer agent.

The active principle can be dissolved into the liquid and filter sterilized before of the distribution into vials or it can be suitably sterilized, being therefore added vials of liquid for injections in the packagings to restore the solution before use.

A local anaesthetic, when necessary, can be added to the excipents both in the case of suppositories and of vials formulations.

The unit dose for the above described formulations wil range from 150 to 300 mg of active principle (even up to 500 mg for suppositories).

For the preparation of formulations for topical use, for creams or unguents fatty base excipients such as vaseline, vaseline oil, lanoline etc. or self-emulsifiable excipients such as alcohols, fats, polyethyleneglycols, ethers or fatty acids esters can be used or other tensides emulsified in water in the case of unguents, ointments or creams.

On the contrary, in the case of preparation of gels of hydrophilic colloids, polymers of various kind will be used, such as carboxyvinylpolymers, sodium carboxymethylcellulose, methylcellulose, Methocel® gelled in water, ethanol, propyleneglycols, glycerol, polyethyleneglycols, etc.

The above mentioned topical preparation can be advantageously added to suitable antibactericides such as Parabens®, phenol derivatives, quaternary ammonium salts etc.

The active principle concentration will range in these formulations from 1 to 5%.

Some compositions are hereinafter reported, by way of examples.

Formulations in tablets, dosed at 250 mg of active principle:

| | |
|---|---|
| Compound I . HCl | 250 mg |
| Microcrystalline cellulose (Avicel®) | 20 mg |
| Lactose | 42 mg |
| Polyvinylpyrrolidone (PVP) | 6 mg |
| Magnesium stearate | 3 mg |
| $SiO_2$ | 1 mg |

Granules of active principles are prepared with Avicel®, lactose and an alcoholic solution of PVP; the granulate obtained is dried, then mixed with magnesium stearate and silicon dioxide and the mixture so obtained is pressed in tablets containing each 250 mg of active principle.

Formulations in capsules, dosed at 250 mg of active principle:

| | |
|---|---|
| Compound I . HCl | 250 mg |
| Maize starch | 100 mg |
| Lactose | 100 mg |
| PVP | 6 mg |
| Magnesium stearate | 5 mg |

The raw materials are sieved, charged in mixer for powders and the mixture is homogenized.

The homogeneous mixture so obtained is distributed in hard gelatine capsules or opercolates by means of filling machine.

Formulations in suppositories dosed at 375 mg of active principle:

| | |
|---|---|
| Compound I | 375 mg |
| Colloidal silica | 8 mg |
| Semisynthetic glycerides (Witepol®) up to | 2000 mg |

The excipient mass is melted at 40°C.

The active principle is mixed with the molten mass by means of a suitable mechanical dispensing apparatus. The mass is cooled to 36°C and cast, keeping under stirring the suppository mass, the PVC or aluminium valves.

The mass is allowed to solidify and the containers are suitably sealed.

Formulations in cream dosed at 5% of active principle:

| | |
|---|---|
| Compound I | 5 g |
| Octyldodecanole (Eutanol G®) | 7 g |
| Liquid $C_8$ triglyceride (Miritol 318®) | 3 g |
| Polyoxyethylene cetostearilic alcohol (Emulgin $B_1/B_2$®) | 2 g |
| Propylene glycol | 5 g |
| Carboxyvinylpolymer (Carbopol 940®) | 1 g |
| Fenocombin® | 1 g |
| Sodium hydroxide to | |
| Distilled water to | 100 g |

The carboxyvinylpolymer is dispersed in water (20% of the amount necessary for the batch preparation) and is neutralized with sodium hydroxide in the amount required to obtain a pH of 5.5.

The fatty phase components are collected in a suitable melter and are melted at the temperature of 70°C.

The active principle is dispersed in glycol and water (10% of the amount necessary for the batch preparation), the preservative agent is dissolved in the residual amount of water and the solution is heated to 80°C.

The aqueous phase is poured in the fatty phase by carrying out the homogenization by means of a suitable emulsifying apparatus. The mixture is cooled at 40°C and the hydroglycolic suspension of the active principle is added to the emulsion.

Finally the emulsion is stabilized with the CVPolymer gel added and dispersed by means of suitable mechanical stirrer.

The pH is checked and adjusted to 5.5.

The cream is distributed in flexible aluminium tubes or other suitable packaging material for topical use preparations.

**Claims for the Contracting States: BE CH DE FR GB IT LI LU NL SE**

1. A compound having formula I

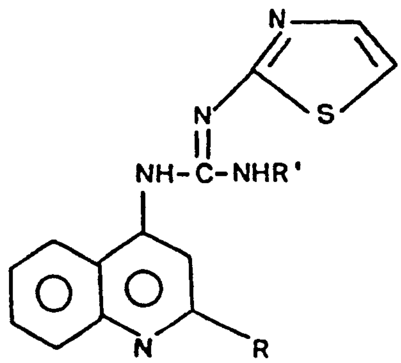

I

in which:

R represents a methylthio group;

R' represents a cyclohexyl group.

2. A process for the preparation of the compounds of formula I consisting of:

a) reacting a 2-methylthio-4-amino-quinoline and cyclohexyl-isocyanate in the presence of bases and in anhydrous medium;

b) dehydrating the urea IV obtained in a) to carbodiimide V, by treatment with triphenylphosphine in carbon tetrachloride in the presence of triethylamine;

c) reacting the carbodiimide V with 2-aminothiazole in anhydrous aromatic hydrocarbon solvents at the reflux temperature.

3. Pharmaceutical compositions having antiinflammatory, analgesic, antipiretic activity containing as active principle therapeutically effective amounts of the compound of formula I or equivalent amounts of its salts, in combination with possible carriers or diluents.

4. Pharmaceutical compositions according to claim 3 for oral, rectal or parenteral administrations, in form of capsules, optionally coated tablets, suppositories or vials, containing from 100 to 500 mg of active principle per unit dose.

5. Pharmaceutical compositions according to claim 3 for topical administration in form of creams, ointments or gels containing from 1% to 5% of the active principle.

**Claims for the Contracting State: AT**

1. Process for the preparation of a compound having formula I

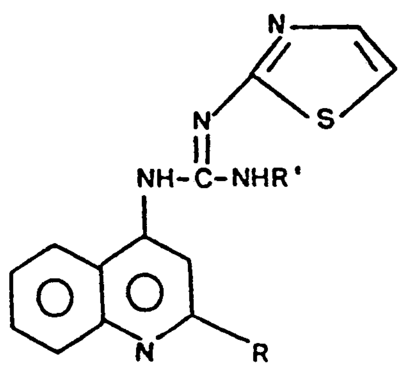

I

in which:

R represents a methylthio group;

R' represents a cyclohexyl group,

which consists of:

a) reacting a 2-methylthio-4-amino-quinoline and cyclohexyl-isocyanate in the presence of bases and in anhydrous medium;

b) dehydrating the urea IV obtained in a) to carbodiimide V, by treatment with triphenylphosphine in carbon tetrachloride in the presence of triethylamine;

c) reacting the carbodiimide V with 2-aminothiazole in anhydrous aromatic hydrocarbon solvents at the reflux temperature.

2. Process according to claim 1 characterized in that toluene is used as anhydrous aromatic hydrocarbon.

**Patentansprüche für die Vertragsstaaten: BE CH DE FR GB IT LI LU NL SE**

1. Verbindung, die die Formel I besitzt:

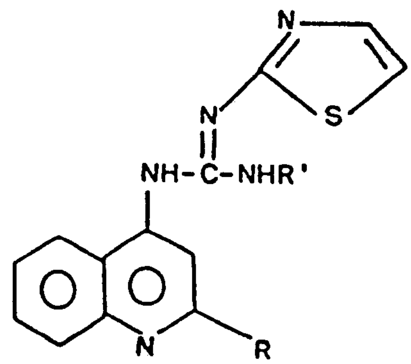

I

wobei

R eine Methylthio-gruppe darstellt,

R′ eine Zyklohexyl-gruppe darstellt.

2. Verfahren zum Herstellen von Verbindungen de Formel I, das folgende Schritte enthält:

a) Reaktion eines 2-Methylthio-4-Amino-Chinolins und Zyklohexyl-Isozyanates bei Vorhandensein von Basen und in einem wasserfreien Medium;

b) Dehydration des Harnstoffes IV, den man in a) gewinnt, zu Carbodiimid V durch Behandlung mit Triphenylphosphin in Kohlenstofftetrachlorid bei Vorhandensein von Triäthylamin;

c) Reaktion des Carbodiimides V mit 2-Aminothiazol in wasserfreien aromatischen Kohlenwasserstofflösemitteln bei Refluxtemperatur.

3. Pharmazeutische Zusammensetzungen, die antientflammbare, analgetische und fiebersenkende Wirkungen aufweisen und die als aktiver Grundbestandteil therapeutisch wirksame Mengen der Verbindung der Formel I oder äquivalente Mengen ihrer Salze in Verbindung mit Trägerstoffen oder Verdünnungsmitteln aufweisen.

4. Pharmazeutische Zusammensetzungen nach Anspruch 5 zur oralen, rektalen oder parenteralen Verabreichung in Form von Kapseln, fakultativ überzogenen Tabletten, Zäpfchen oder Ampullen, die 100—500 mg eines aktiven Grundstoffes pro Dosiseinheit enthalten.

5. Pharmazeutische Zusammensetzungen nach Anspruch 3 zur äußerlichen Anwendung in Form von Cremes, Salben oder Gelen, die 1—5% ds aktiven Grundstoffes enthalten.

**Patentansprüche für den Vertragsstaat: AT**

1. Verfahren zur Herstellung der Verbindung, die die Formel I besitzt:

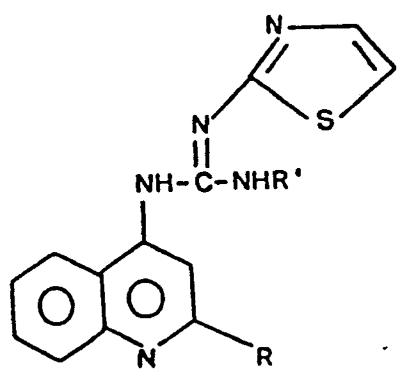

I

wobei

R eine Methylthio-gruppe darstellt,

R′ eine Zyklohexyl-gruppe darstellt wobei das Verfahren aus folgenden Schritten besteht:

a) Reaktion eines 2-Methylthio-4-Amino-Chinolins und Zyklohexyl-Isozyanates bei Vorhandensein von Basen und in einem wasserfreien Medium;

b) Dehydration des Harnstoffes IV, den man in a) gewinnt, zu Carbodiimid V durch Behandlung mit Triphenylphosphin in Kohlenstofftetrachlorid bei Vorhandensein von Triäthylamin;

c) Reaktion des Carbodiimides V mit 2-Aminothiazol in wasserfreien aromatischen Kohlenwasserstofflösemitteln bei Refluxtemperatur.

2. Verfahren nach Anspruch 1, dadurch gekennzeichnet, daß als wasserfreier aromatischer Kohlenwasserstoff Toluol verwendet wird.

**Revendications pour les Etats contractants: BE CH DE FR GB IT LI LU NL SE**

1. Composé ayant la formule I

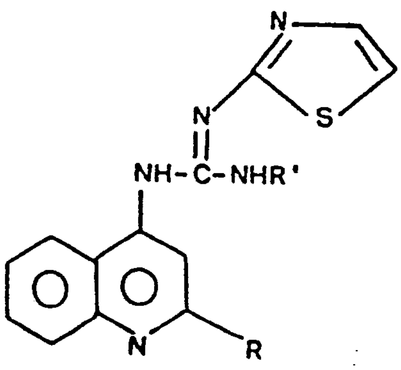

I

où

R est un groupe méthylthio;

$R^1$ est un groupe cyclohexyle.

2. Procédé de préparation des composés de formule I, qui consiste à

a) faire rèagir une 2-méthylthio-4-amino-quinoléine et du cyclohexyl-isocyanate en présence de bases et dans un milieu anhydre;

b) déshydrater l'urée IV obtenue en a) en carbodiimide V, par traitement avec de la triphénylphosphine dans du tétrachlorure de carbone en présence de triéthylamine;

c) faire réagir le carbodiimide V avec du 2-aminothiazole dans des solvants hydrocarbures aromatiques anhydres à la température de reflux.

3. Compositions pharmaceutiques ayant une activité anti-inflammatoire, analgésique et antipyrétique, contenant comme principe actif des quantités thérapeutiquement actives du composé de la formule I ou des quantités équivalentes de ses sels, en combinaison avec d'éventuels véhicules ou diluants.

4. Compositions pharmaceutiques selon la revendication 3 pour l'administration orale, rectale ou parentérale sous forme de gélules, de comprimés éventuellement revêtus, de suppositoires ou d'ampoules, contenant 100 à 500 mg de principe actif par dose unitaire.

5. Compositions pharmaceutiques selon la revendication 3 pour l'administration topique sous forme de crèmes, onguents ou gels, contenant de 1 à 5% de principe actif.

**Revendications pour l'Etat contractant: AT**

1. Procédé de préparation du composé ayant la formule I

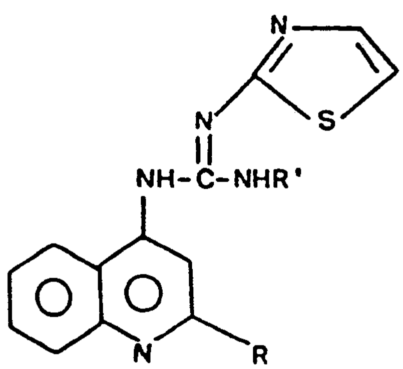

I

où

R est un groupe méthylthio;

$R^1$ est un groupe cyclohexyle,
qui consiste à

a) faire rèagir une 2-méthylthio-4-amino-quinoléine et du cyclohexyl-isocyanate en présence de bases et dans un milieu anhydre;

b) déshydrater l'urée IV obtenue en a) en carbodiimide V, par traitement avec de la triphénylphosphine dans du tétrachlorure de carbone en présence de triéthylamine;

c) faire réagir le carbodiimide V avec du 2-aminothiazole dans des solvants hydrocarbures aromatiques anhydres à la température de reflux.

2. Procédé selon la revendication 1 dans lequel le toluène est utilisé comme hydrocarbure aromatique anhydre.